# EUROPEAN PATENT APPLICATION

(11) **EP 4 434 441 A1**
(43) Date of publication of application: **25.09.2024**
(21) Application number: 23162812.4
(22) Date of filing: 20.03.2023
(51) Int. Cl.: A61B 5/00, A61B 5/11, A61B 5/332

(54) **ANXIETY-AVOIDING METHOD AND SYSTEM FOR THE RECORDING OF HEALTH-RELATED SYMPTOMS AND ACTIVITIES**

(71) Applicant: Koninklijke Philips N.V., 5656 AG Eindhoven (NL)
(72) Inventor: STUT, Wilhelmus Johannes Joseph, 5656AG Eindhoven (NL); EJUPI, Andreas, 5656 AG Eindhoven (NL)
(74) Representative: Philips Intellectual Property & Standards

(57) **Abstract**

A system (**1**) includes a monitoring device (**10**) including at least one sensor (**12, 14**), the monitoring device configured to be worn by an associated patient and the at least one sensor configured to measure physiological sensor data (**13, 15**) of the associated patient wearing the monitoring device. An electronic processor (**16**, **21**) is integrated with or in wireless communication with the monitoring device. The electronic processor is programmed to analyze the physiological sensor data to detect a health-related event; and prompt the associated patient to provide information via a user interface (**24**) based on the detected health-related event.

## Description

The following relates generally to the medical monitoring arts, wearable medical monitor arts, heart rate monitoring arts, patient activity monitoring arts, and related arts.

### BACKGROUND

Health-related unobtrusive sensing systems enable replacement of continued hospitalization with obtrusive vital signs sensor technologies, centered around the individual, to provide remote monitoring of the subject's general health condition. Vital signs monitoring typically includes monitoring one or more of the following physical parameters: heart rate (HR), blood pressure (BP), respiratory rate (RR), core body temperature and blood oxygenation (SpO₂).

Current ambulatory ECG monitors and mobile cardiac telemetry systems gather ECG data in ambulatory settings (e.g. in the patient's home environment or on-the-go). In such systems, a sensor is placed in a patch on the chest. The sensor communicates wirelessly with a dedicated smartphone which is connected via the Internet to the clinical service center. In other embodiments, the sensor may be directly connected to the Internet. Some examples of such devices include the ePatch (Extended Holter Monitor) and MCOT (Mobile Cardiac Telemetry) devices available from Philips ECG Solutions. These devices provide a wearable single-use electrodes patch onto which an electronics module attaches to form a patient-worn device.

In such systems, when patients feel a symptom, they can inform the sensor about the occurrence of a symptom by touching it in a specific way (e.g., double-tapping the sensor, a dedicate button on the sensor, and so forth). Touching the sensor in a specific way adds a mark to the ECG data stream. By touching the sensor, the sensor only registers that a symptom has occurred, but the patient has not described or recorded the symptoms and activity yet.

The description of the symptom and the activity is typically done via an app on a smartphone of the patient. When patients feel a symptom, they can select the symptom recording function of the app, and provide details like the nature of the symptom (e.g. fainting, dizziness, chest pain, and so forth), the activity during which the symptom occurred (e.g. walking, cycling), and/or the (perceived) intensity of the activity (e.g. light, medium, heavy, etc.).

The drawback of traditional patient symptom records is that they are often inaccurate or incomplete. When patients experience a symptom, they may simply forget to describe the activity and symptom, or even ignore it. Furthermore, so-called "silent" cardiac events cannot be noticed since they (by definition) involve no symptoms at all, which means that the patient even does not know s/he should describe the activity.

In addition, when the ECG system detects a cardiac event, it could ask the patient to record their activity and symptom. However, this approach may lead to patient anxiety and uncertainty, especially when the event is a silent cardiac event.

The following discloses certain improvements to overcome these problems and others.

### SUMMARY

In one aspect, a system includes a monitoring device including at least one sensor, the monitoring device configured to be worn by an associated patient and the at least one sensor configured to measure physiological sensor data of the associated patient wearing the monitoring device. An electronic processor is integrated with or in wireless communication with the monitoring device. The electronic processor is programmed to analyze the physiological sensor data to detect a health-related event; and prompt the associated patient to provide information via a user interface based on the detected health-related event.

In another aspect, a method of monitoring an associated patient includes analyzing physiological sensor data of a patient obtained by a monitoring device to detect a health-related event; and prompting the associated patient to provide information via an associated mobile device based on the detected health-related event.

One advantage resides in providing a reliable remote monitoring system for monitoring a patient.

Another advantage resides in ensuring accurate patient symptoms records.

Another advantage resides in prompting patients to describe their symptoms based on collected physiological data of the patient.

Another advantage resides in prompting patients at random times to describe their symptoms.

Another advantage resides in reducing patient anxiety and uncertainty in reporting their symptoms.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
Fig. 1 diagrammatically shows a patient monitoring system in accordance with the present disclosure.
Fig. 2 shows an example flow chart of operations suitably performed by the system of Fig. 1.
Fig. 3 shows an example of a process performed by the system of Fig. 1.
Fig. 4 shows an example of data collected by the system of Fig. 1.

### DETAILED DESCRIPTION

As used herein, the singular form of "a," "an", and "the" include plural references unless the context clearly dictates otherwise. As used herein, statements that two or more parts or components are "coupled," "connected," or "engaged" shall mean that the parts are joined, operate, or co-act together either directly or indirectly, i.e., through one or more intermediate parts or components, so long as a link occurs. Directional phrases used herein, such as, for example and without limitation, top, bottom, left, right, upper, lower, front, back, and derivatives thereof, relate to the orientation of the elements shown in the drawings and are not limiting upon the scope of the claimed invention unless expressly recited therein. The word "comprising" or "including" does not exclude the presence of elements or steps other than those described herein and/or listed in a claim. In a device comprised of several means, several of these means may be embodied by one and the same item of hardware.

The disclosed system aims to avoid anxiety by not only asking patients about their symptoms and activity when the system has detected a cardiac event, but also at additional moments. When the patient starts using the disclosed system, the patient will be explained that the disclosed system can ask him/her to describe symptoms and activities after a cardiac event, but also at other moments. Hence, since a request to describe symptoms and activities does not imply that the patient just had a cardiac event, this approach is assumed to reduce (or even prevent) patient anxiety and uncertainty.

The moments at which the system sends these recording requests, depend amongst others on the detected cardiac events, time of the day, and previous spontaneous recording behavior of patients. Furthermore, when the disclosed system has an accelerometer to detect the activity level over time, it may ask patients to provide symptom and activity information based on the observed activity level or activity pattern, possibly in combination with previous spontaneous recording behavior of patients. In both cases the system learns from previous spontaneous recording behavior of patients and triggers new reporting requests based on similar characteristics.

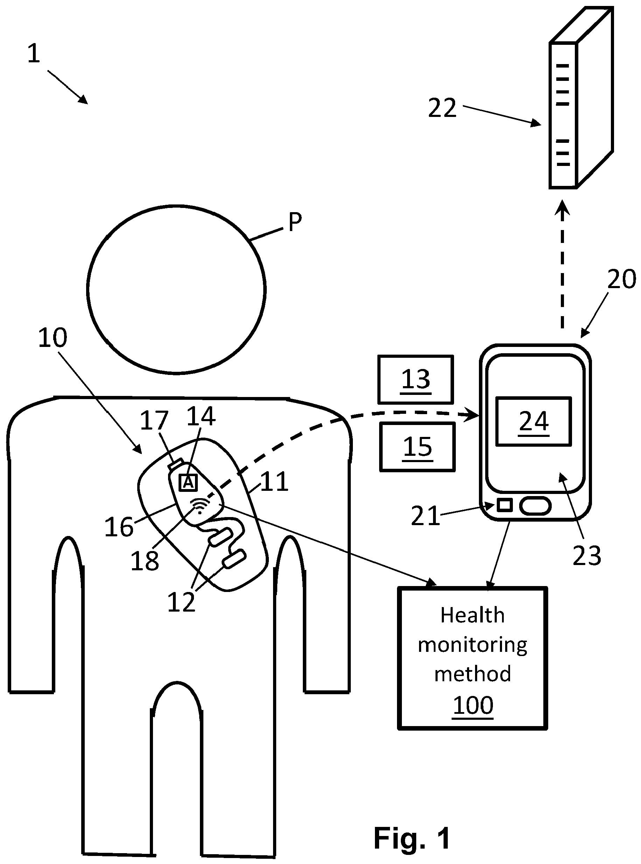

With reference to Fig. 1, a system **1** for monitoring an associated patient **P** is shown. As used herein, the term "patient" (and variants thereof) refers to, and includes, an outpatient, a discharged patient, a patient undergoing screening using the monitoring device as part of an annual medical checkup, or other person whose health condition is to be monitored. As shown in Fig. 1, the system **1** includes a wearable monitoring device **10** that is wearable by, or otherwise attached to, the patient **P.** The wearable monitoring device **10** can include any suitable monitoring device, such as a Mobile Cardiac Outpatient Telemetry (MCOT)^{®} device (available from Philips ECG Solutions, Malvern, Pennsylvania, USA), or a medical wearable device, a torso-worn vital signs health patch, a wrist-worn watch, a chest strap, a smart garment, medical ear buds/over the ear, a forehead or nose sensor, a smart ring, or so forth.

The illustrative wearable monitoring device **10** includes a single-use electrodes patch **11** with an ECG sensor **12** (e.g., embodied as electrodes **12)** for collecting cardiac data **13,** and an accelerometer **14** collecting accelerometer data **15.** The illustrative monitoring device **10** includes an electronics module **16** that attaches onto the electrodes patch **11,** and the accelerometer **14** may be integrated into the electronics module **16.** The electronics module **16** includes at least one microprocessor or microchip (not shown) configured (e.g. programmed) to optionally preprocess the ECG signals from the ECG sensor (electrodes) **12** to produce the cardiac data **13** and/or optionally preprocess the data from the accelerometer **14** to produce the accelerometer data **15.** The device **10** including the electrodes patch **11** and the attached electronics module **16** is adhesively secured to the chest or other anatomy of the patient **P** after suitable preparation (e.g. cleaning and/or shaving) of the skin. The use of the separate electrodes patch **11** and electronics module **16** advantageously enables the single-use electrodes patch **11** to be a low-cost component that can be replaced as needed over the course of a patient monitoring session (which may extend over multiple days or weeks) while re-using the more expensive electronics module **16.** However, other arrangements are contemplated such as having the patch **11** and electronics **16** constructed as a unitary single-use unit. Although not shown, it will be appreciated that the wearable monitoring device **10** also includes an on-board battery or other on-board electrical power source to power the electronics module **16.** The on-board battery may, for example, be integrated with the electronics module **16,** which may include a recharging port connector **17** for recharging the electronics module **16,** or the electronics module **16** could be placed on a wireless inductive recharging station to recharge it if needed during a patient monitoring study.

The wearable monitoring device **10** more generally can include one or more sensors configured to measure physiological sensor data of the patient **P** wearing the monitoring device **10.** The measured physiological sensor data of the patient **P** is used to detect a health-related event of the patient **P.** As shown in Fig. 1, the monitoring device **10** includes two sensors - an electrocardiogram (ECG) sensor **12** and an accelerometer **14.** The ECG sensor **12** is configured (e.g., comprising skin-contacting electrodes with silver/silver chloride coatings, for example) to measure cardiac data **13** of the patient **P,** and the health-related event comprises a cardiac event detected by analysis of the cardiac data **13.** In addition, the ECG sensor **12** can be replaced with (or supplemented by) any other suitable sensor to measure a corresponding vital sign of the patient (e.g., SpO₂, electroencephalogram (EEG), and so forth). The accelerometer **14** is configured to measure patient activity data of the patient **P,** more specifically, accelerometer data **15.**

The wearable monitoring device **10** also includes a wireless transmitter or transceiver **18** (referred to hereinafter as a transceiver **18),** which may optionally be integrated with the electronics module **16.** The transceiver **18** is integrated with or in wireless communication with the monitoring device **10** to transmit the patient data (e.g. the cardiac data **13** and accelerometer data **15)** to a mobile device **20.** In some embodiments, the electronic processor **16** is configured to collect and optionally preprocess the cardiac data **13** from the ECG sensor **12** and/or the accelerometer data **15** from the accelerometer **14,** and the transceiver **18** is configured to transfer the cardiac data **13** and/or the accelerometer data **15** to the mobile device **20** (e.g., a cellphone or other smart device, or a dedicated medical monitoring device) operable by the patient **P.** In a typical arrangement, the transceiver **18** is a low-power wireless transceiver (e.g., Bluetooth^{™}, Zigbee^{™}, or the like) that connects with low power to the mobile device **20,** thus placing a low power draw on the on-board battery of the wireless monitoring device **10.** The patient **P** can be prompted to provide information, including at least health-related symptoms that the patient **P** is experiencing, using the mobile device **20** running an application ("app") executed by an electronic processor **21** of the mobile device **20.** In some examples, this information can be transmitted to a clinical health information system **22** comprising a server computer, for example transmitted over the Internet via a 3G/4G/5G wireless cellular network, Wi-Fi, various combinations thereof, and/or another wireless communication protocol. Using the intermediary mobile device **20** has certain advantages such as providing a display **23** on which a user interface (UI) **24** can be displayed, e.g. to present a diary for the patient to record health symptoms. The mobile device **20** can also perform some or all processing of the cardiac and accelerometer data **13** and **15** instead of performing that processing at the on-board electronics module **16** of the wearable monitoring device **10.** However, it is alternatively contemplated to omit the separate mobile device **20** and instead have all processing performed by the electronics module **16** of the wearable monitoring device **10,** and to have the transceiver **18** of the wearable monitoring device **10** wirelessly communicate directly with the clinical health information system **22.** In such embodiments the wearable monitoring device may also include a display for presenting the UI **24** - for example, the wearable monitoring device **10** could have the form factor of a wristwatch.

The processing of the patient data **13** and **15** can be variously distributed between the electronic module **16** of the wearable monitoring device **10** and the mobile device **20.** Hence, an electronic processor **16, 21** is referred to herein, to indicate the combined processing capacity of the electronic module **16** of the wearable monitoring device **10** and the mobile device **20.** In general, processing described as being performed by the electronic processor **16, 21** may be performed entirely by the electronic module or processor **16** of the wearable monitoring device **10,** or entirely by the electronic processor **21** of the mobile device **20,** or the processing may be shared between them.

As further diagrammatically indicated in Fig. 1, the electronic processor **16, 21** can be configured to perform a health status monitoring method **100** of monitoring the patient **P** by analyzing the cardiac data **13** and/or the accelerometer data **15.**

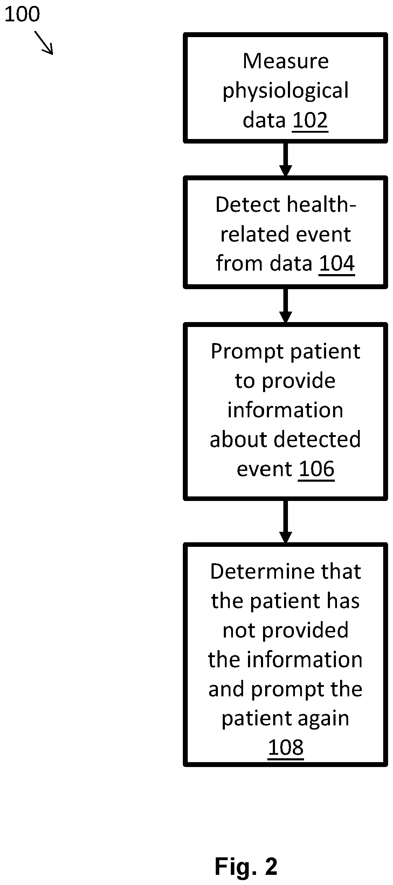

With reference now to Fig. 2, an illustrative embodiment of the health status monitoring method **100** is shown by way of a flowchart. To begin the method **100,** the monitoring device **10** is attached to the patient **P.** At an operation **102,** the cardiac data **13** is measured by the ECG sensor **12** and the accelerometer data **15** is measured by the accelerometer **14.** At an operation **104,** the electronic processor **16, 21** is configured to analyze the cardiac data **13** to detect a health-related event of the patient **P** (and the accelerometer data **15** to detect an activity-related event of the patient **P).** At an operation **106,** the electronic processor **16, 21** is programmed to prompt the patient **P** to provide information via the mobile device **20** based on the detected health-related event. The patient **P** providing the information is also sometimes referred to herein as making a diary entry. To do so, the electronic processor **16, 21** is configured to control the mobile device **20** to display the user interface (UI) **24** on a screen of the mobile device **20.** The UI **24** is configured to receive inputs from the patient **P** to input the information. In some embodiments, the UI **24** may be a graphical user interface (GUI). In some embodiments, the UI **24** provides the prompt by presenting a diary entry UI comprising a set of user dialogs with symptoms listed (e.g., fainted, dizzy, chest pain, light headed, skipped heartbeat, shortness of breath, heart racking, or so forth) which can be selected by the patient by checking associated boxes or the like, along with user-selectable listed activities (resting, light activity, medium activity, heavy activity); and/or may provide a freeform text entry dialog for the user to describe his or her symptoms in greater detail. Upon completion of the diary entry it is suitably transmitted from the mobile device **20** to the clinical health information system **22.**

In general, the patient can initiate a diary entry manually, for example by selecting a "Make diary entry" button or the like shown on the UI **24.** For example, the patient's physician or the supplier of the system 1 will instruct the patient to initiate a diary entry any time the patient experiences a symptom. This provides valuable contextual information for consideration by the patient's physician when reviewing the patient data collected by the patient monitoring session.

However, analysis of the cardiac data **13** by the electronic processor **16, 21** can detect certain types of cardiac events that may not be perceived by the patient. These are sometimes referred to as silent cardiac events. More generally, physiological sensor data from the monitoring device **10** can be analyzed by the electronic processor **16, 21** to detect a health-related event that might not be perceived by the patient. As another example, an EEG monitor may detect abnormal brain activity that is not consciously recognized by the patient. Hence, reliance on manually initiated diary entries will not be effective to obtain contextual information for such silent health symptoms. Moreover, even if the patient feels a symptom he or she may fail to proactively initiate a diary entry in a timely fashion to provide the contextual information. The operation **104** of the health monitoring method **100** advantageously detects silent health-related events or health-related events that are ignored by the patient, and automatically initiates the prompt **106.**

The operations **104** and **106** can be performed in a variety of manners. In particular, the electronic processor **16, 21** is programmed to additionally prompt the patient **P** to provide the information via the mobile device **20** at times not associated with a detected cardiac event. In one example, the electronic processor **16, 21** is configured to prompt the patient **P** to provide the information via the mobile device **20** at a random time independent of whether a health-related event was detected. As used herein, the term "random" can comprise a completely random or a pseudorandom occurrence that can be deterministic, but not as regular intervals. Advantageously, this approach compensates for that fact cardiac events are often silent in combination with the problem of making the patient anxious about reporting their symptoms. In other words, if the patient **P** knows or suspects that the system **1** prompted a diary entry due to detection of a silent cardiac event, the very fact of the prompt can induce anxiety in the patient **P.** By additionally making random prompts that are unrelated to detected silent cardiac events, the patient **P** comes to expect prompts to occur on occasion, and can be accurately informed that the diary entry prompts are not usually related to silent cardiac events. To do so, the electronic processor **16, 21** is configured to detect, from measured activity data **15** of the patient **P,** a sleeping time period during which the patient **P** is sleeping, and not prompt the patient **P** to provide the information during the determined sleeping time period. In another example, the electronic processor **16, 21** is configured to detect, from measured activity data **15** of the patient **P,** a period of activity (e.g., running, walking, exercising, etc.) and/or inactivity (e.g., laying down, watching television, etc.), and at the end of the activity session (or during the inactivity session), prompt the patient **P** to provide the information via the mobile device **20.** These are merely examples, and should not be construed as limiting.

At an operation **108,** the electronic processor **16, 21** is programmed to determine whether the patient **P** has provided information via the mobile device **20** describing the detected health-related event, and the prompt to provide information based on the detected health-related event is further based on it being determined that the patient **P** has not provided the information.

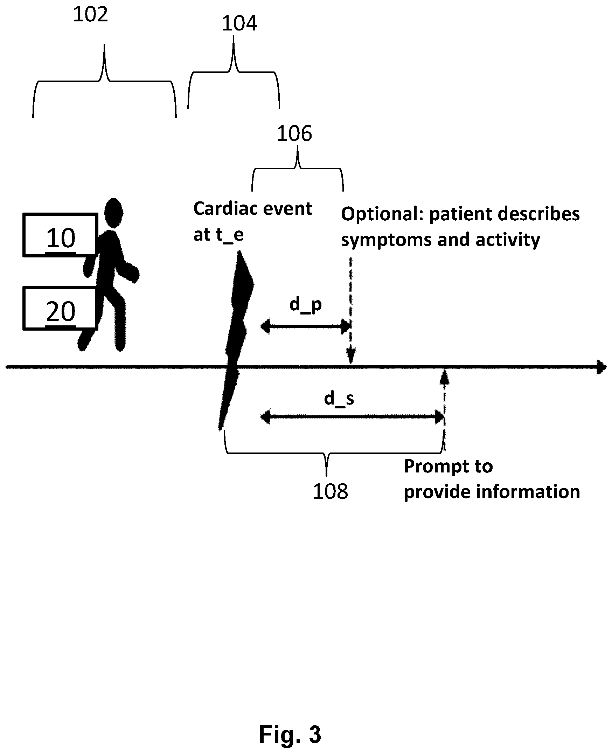

Fig. 3 shows the operations of the method **100** as a timeline. The operation **102** is shown with the patient **P** wearing the monitoring device **10** and having the mobile device **20** on their person, and the physiological data **13, 15** is collected. Based on the physiological data **13, 15,** the monitoring device **10** detects that at time *t_e* (time of event) a cardiac event has occurred (i.e., the operation **104).** At the operation **106,** after a delay of *d_p* (i.e., a delay by the patient **P)** the patient **P** describes the symptom and activity on their own via the mobile device **20.** At the operation **108,** after a delay of *d_s* (i.e., a delay by the system 1) the monitoring device **10** checks whether the patient **P** has described the health-related event. If not, at a time *t_e* + *d_s,* the monitoring device **10** asks the patient **P** to describe the symptom and activity around *t_e* by inputting the information to the mobile device **20.** The value of *d_s* can be fixed (e.g. 5 minutes), but can also depend on the spontaneous recording behavior of the patient, i.e. the observed *d_p* values of the patient **P.** Example values for *d_s* are, for example, *d_s* = *average (*observed *d_p values)* + *2 minutes, d_s* = *average (observed d_p values)* * 2, and *d_s* = *average (observed d_p values)* + *standard deviation (observed d_p values).* The system **1** may also let the value of *d_s* depend on the observed *d_p* values in other patients. Especially in the first hours or days of the monitoring period, when the patient **P** had no cardiac events yet (and therefore no *d_p* values), this is an attractive approach.

To prevent patient anxiety, the system **1** not only asks patients about their symptoms and associated activity when it has detected a cardiac event, but also at so-called *additional moments.* Example additional moments are, for example, randomly selected moments (e.g. random moments between 8 a.m. and 10 p.m.), fixed moments (e.g. 8 a.m., 2 p.m., and 10 p.m.), a random delay after the last symptom and activity recording (e.g. 4 to 6 hours later), a moment in time similar to previous moments where symptoms were reported (e.g. before lunch or bed time), and so forth. The system **1** should not do this when the patient **P** has just reported symptoms and activity, or when the system **1** has just detected a cardiac event at *t_e* and is still waiting until *t_e* + *d_s.* In that case, a next additional moment will be determined.

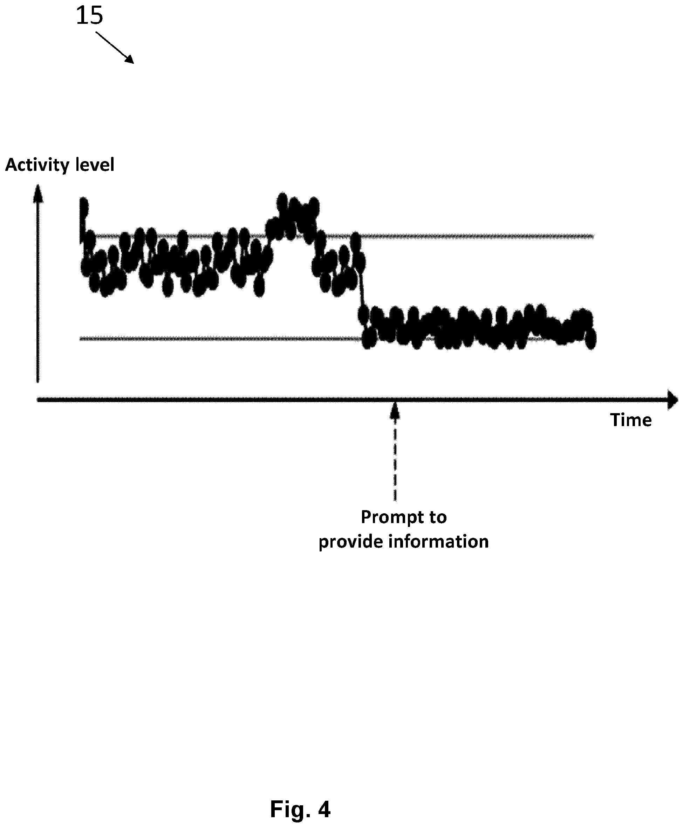

Fig. 4 shows an example of the accelerometer data **15** being used to detect the activity-related events of the patient **P.** The accelerometer data **15** can be used to detect activity sessions (e.g. walking or running sessions). When an activity session has ended, the system **1** can ask the patient **P** whether they had symptoms during this activity session, and what type of activity (e.g. walking, running, cycling) they were doing, as shown in Fig. 4. In some examples, the activity type information can also be used to train a machine learning algorithm for automated activity type detection.

The accelerometer **15** can also be used to detect periods of inactivity (e.g. the patient has been sitting for a longer period) where it is more likely that a patient **P** has time to report and reacts calmly to a reporting request. Furthermore, information from the mobile device **20** (e.g. a phone in use) and/or other sensors (e.g. low heart rate from ECG signal) can be used/added to determine these periods of inactivity. Some examples can include, for example, a low heart rate from ECG signal, a low heart rate from ECG signal AND phone not in use, and so forth. The accelerometer **14** can also be used to detect activity patterns preceding spontaneous reporting. For example, the system **1** may notice that a patient **P** always spontaneously reports symptoms after a night of disturbed sleep. If such an activity pattern occurs again, but the patient **P** has not spontaneously reported symptoms and activity afterwards, the system **1** may ask the patient **P** to report symptoms and activity.

When asking the patient **P** about their symptoms and associated activity (i.e., after a cardiac event or at an additional moment), the system 1 should first ask whether the patient **P** experienced symptoms at a particular moment or during a particular period. Only when the patient **P** confirms, the UI **24** to describe the symptoms will be shown. The UI **24** to describe the activity are always shown. The request to describe symptoms and activity may refer to the present (i.e. the current moment or the past *n* minutes), or to the past (i.e. a moment or a period in the past). When the system **1** asks the patient **P** to describe their symptoms and activity, it may send a notification (e.g. a push notification on the mobile device **20)** to draw their attention.

In some examples, the patient **P** may be sleeping when the system **1** asks to record symptoms and activity. To prevent that the system **1** disturbs the sleeping patient **P,** the system **1** can be configured to not send puh notifications when the patient **P** is sleeping (e.g. detected via the accelerometer data **15)** or during configurable time zones (e.g. 10:00 PM to 08:00 AM). The system **1** can be configured to not present the UI **24** when the patient **P** is sleeping or during configurable time zones (e.g. 10:00 PM to 08:00 AM). In the latter case, the accelerometer **14** can be used to determine when the patient **P** got out of bed in the morning. After a configurable delay (e.g. 15 minutes after getting out of bed), the system **1** may ask whether the patient **P** felt symptoms, for example, during the past night (i.e., without indicating a specific moment) or at specific moments during the night (e.g. at 03:40 a.m.). These moments may correspond to detected cardiac events, or be additional moments.

The system **1** may also ask the patient to take additional measurements (e.g. with an SpO2 sensor) after a cardiac event or at additional moments. In another example, a number of requests to report symptoms and activity may be bound to a (i.e., daily) limit. Different limits may apply to the number of requests after cardiac events, the number of additional moments, or the sum thereof.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. A system **(1)** comprising:
a monitoring device **(10)** including at least one sensor **(12, 14),** the monitoring device configured to be worn by an associated patient and the at least one sensor configured to measure physiological sensor data **(13, 15)** of the associated patient wearing the monitoring device;
an electronic processor **(16, 21)** integrated with or in wireless communication with the monitoring device, the electronic processor programmed to:
analyze the physiological sensor data to detect a health-related event; and
prompt the associated patient to provide information via a user interface **(24)** based on the detected health-related event.

2. The system **(1)** of claim 1, wherein the electronic processor **(16, 21)** is programmed to:
prompt the patient to provide the information via the user interface **(24)** at a random time independent of whether a health-related event was detected.

3. The system **(1)** of claim 2, wherein the electronic processor **(16, 21)** is further programmed to:
detect, from measured activity data of the associated patient, a sleeping time period during which the associated patient is sleeping;
wherein the electronic processor is programmed to not prompt the associated patient to provide the information during the sleeping time period.

4. The system **(1)** of claim 2, wherein the electronic processor **(16, 21)** is further programmed to additionally prompt the associated patient to provide the information via the user interface **(24)** at times not associated with a detected health-related event.

5. The system **(1)** of any one of claims 1-4, wherein the at least one sensor **(12, 14)** comprises an electrocardiogram (ECG) sensor **(12)** configured to measure cardiac data of the associated patient, and the health-related event comprises a cardiac event detected by analysis of the cardiac data.

6. The system **(1)** of any one of claims 1-5, wherein the electronic processor **(16, 21)** is integrated with the monitoring device **(10),** and the monitoring device further includes a wireless transmitter or transceiver **(18)** and is disposed on a patch attachable to the associated patient.

7. The system **(1)** of any one of claims 1-6, wherein the at least one sensor **(12, 14)** comprises an accelerometer **(14)** configured to measure activity data of the associated patient, and the on-board electronic processor **(16, 21)** is programmed to:
analyze the activity data to detect an activity-related event.

8. The system **(1)** of claim 7, wherein the monitoring device **(10)** includes an ECG sensor **(12),** the accelerometer **(14),** the electronic processor **(16, 21),** and a wireless transmitter or transceiver **(18)** disposed on a patch attachable to a portion of the associated patient.

9. The system **(1)** of any one of claims 1-8, wherein the electronic processor **(16, 21)** is programmed to:
determine whether the associated patient has provided information via the associated mobile device **(20)** describing the detected health-related event; and
wherein the prompt to provide information based on the detected health-related event is further based on it being determined that the associated patient has not provided the information.

10. The system **(1)** of any one of claims 1-9, wherein the at least one sensor **(12, 14)** comprises an accelerometer **(14)** configured to measure activity data of the associated patient, and the electronic processor **(16, 21)** is programmed to:
detect, from the measured activity data, a time of an activity session of the associated patient; and
at an end of the activity session, prompt the associated patient to provide the information via the associated mobile device **(20).**

11. The system **(1)** of any one of claims 1-10, wherein the at least one sensor **(12, 14)** comprises an accelerometer **(14)** configured to measure activity data of the associated patient, and the electronic processor **(16, 21)** is programmed to:
detect, from the measured activity data, a time of an inactivity session of the associated patient; and
during the inactivity session, prompt the associated patient to provide the information via the associated mobile device **(20).**

12. The system **(1)** of any one of claims 1-11, wherein the information includes at least health-related symptoms that the associated patient is experiencing.

13. The system (1) of any one of claims 1-12, wherein the electronic processor **(16, 21)** is programmed to:
control the associated mobile device **(20)** to display a user interface **(24)** configured to receive inputs from the associated patient to input the information.

14. A method **(100)** of monitoring an associated patient, the method comprising:
analyzing physiological sensor data of a patient obtained by a monitoring device **(10)** to detect a health-related event; and
prompting the associated patient to provide information via an associated mobile device (20) based on the detected health-related event.

15. The method **(100)** of claim 14, further including:
prompting the patient to provide the information via the user interface **(24)** at a random time independent of whether a health-related event was detected.
